# EUROPÄISCHE PATENTSCHRIFT

(11) **EP 4 464 501 B1**
(45) Veröffentlichungstag und Bekanntmachung des Hinweises auf die Patenterteilung: **29.10.2025**
(21) Anmeldenummer: 24173376.5
(22) Anmeldetag: 30.04.2024
(51) Int. Cl.: B29C 49/42, B65B 55/02, B65B 55/10, B67C 7/00, C12Q 1/22, G01N 31/22, A61L 2/18, A61L 2/20, B67C 3/22

(54) **BEHANDLUNGSVORRICHTUNG FÜR BEHÄLTER UND/ODER VORFORMLINGE UND VERFAHREN ZUM STEUERN DER BEHANDLUNGSVORRICHTUNG**
TREATMENT DEVICE FOR CONTAINERS AND/OR PREFORMS AND METHOD FOR CONTROLLING THE TREATMENT DEVICE
DISPOSITIF DE TRAITEMENT POUR RÉCIPIENTS ET/OU PRÉFORMES ET PROCÉDÉ DE COMMANDE DU DISPOSITIF DE TRAITEMENT

(30) Priorität: 16.05.2023 DE 102023112866
(43) Veröffentlichungstag der Anmeldung: 20.11.2024
(73) Patentinhaber: KHS GmbH, 44143 Dortmund (DE)
(72) Erfinder: Gerhards, Martin, 44143 Dortmund (DE); Herold, Thomas, 44143 Dortmund (DE)

(56) Entgegenhaltungen:
- EP-A1- 2 354 017
- EP-B1- 1 858 560
- DE-A1- 102016 125 027
- DE-T2- 60 202 362
- DE-T2- 60 204 543
- US-A1- 2022 390 399

## Beschreibung

Die Erfindung betrifft eine Behandlungsvorrichtung für Behälter und/oder Vorformlinge und ein Verfahren zum Steuern der Behandlungsvorrichtung.

Behandlungsvorrichtungen für Behälter und/oder Vorformlinge sind aus US 2022/390399 A1, DE 602 02 362 T2, DE 10 2016 125027 A1, DE 602 04 543 T2, EP 1 858 560 B1 und EP 2 354 017 A1 bekannt. US 2022/390399 A1 offenbart zum Beispiel eine Vorrichtung nach dem Oberbegriff des Anspruchs 1.

Bei der Herstellung und dem Füllen von Behältern, die aus Vorformlingen hergestellt werden, können Anlagen verwendet werden, die Behandlungsvorrichtungen für die Behälter bzw. Vorformlinge, zum Beispiel Streckblasmaschinen, Formfillmaschinen und/oder Füllmaschinen, aufweisen. Die Behandlungsvorrichtungen können für aseptische Verwendungen sterilisiert werden. Dazu werden Sterilisationsmittel in die Behandlungsvorrichtungen eingeführt, die eine Sterilisation von Oberflächen der Behandlungsvorrichtungen bewirken. Für die Sterilisation kann zum Beispiel Wasserstoffperoxid verwendet werden. Die Konzentrationen bei der Sterilisation der Behandlungsvorrichtung von Wasserstoffperoxid in der Raumluft können im Bereich von 1000 ppm bis 5000 ppm liegen. Diese Konzentration von Sterilisationsmittel sind in der Regel sehr gesundheitsschädlich, sodass ein Betreten der Behandlungsvorrichtung durch Bedien-/Wartungspersonal erst nach dem Entfernen der Sterilisationsmittel aus der Behandlungsvorrichtung erfolgen darf.

Die gesetzlichen Grenzwerte für beispielsweise Wasserstoffperoxid, die vor dem Betreten der Behandlungsvorrichtung durch Personal unterschritten werden müssen, liegen meist im Bereich von 0,5 ppm bis 2 ppm. Erst wenn ein Sensor diesen Messwert oder einen kleineren Messwert ausgibt, dürfen Zugänge zu der Behandlungsvorrichtung freigeschaltet werden. Bekannt ist, Sensoren mit einem Messbereich, der diese Werte umfasst, an der Behandlungsvorrichtung zu verwenden.

Daher ist es erforderlich, den Gehalt an Sterilisationsmittel in der Behandlungsvorrichtung zu erfassen, um eine Gesundheitsbeeinträchtigung des Bedien-/Wartungspersonals zu vermeiden. Die Messung von Sterilisationsmittel im Sterilisationsprozess für Behälter ist beispielsweise aus EP 1 858 560 B1 bekannt, wobei die dafür verwendeten Sensoren im Stand der Technik auch zur Überwachung der Konzentration von Sterilisationsmittel innerhalb von Behandlungsvorrichtungen verwendet werden können. Nach einem Absinken der Konzentration des Sterilisationsmittel in der Behandlungsvorrichtung benötigen diese Sensoren jedoch eine vergleichsweise lange Zeitspanne, bis sie korrekte Konzentrationswerte anzeigen, da zunächst das Sterilisationsmittel aus dem Sensor entweichen muss.

Aufgabe der Erfindung ist es, eine Behandlungsvorrichtung für Behälter und/oder Vorformlinge sowie ein Verfahren zum Steuern der Behandlungsvorrichtung bereitzustellen, bei denen nach einer Sterilisation einer Behandlungsvorrichtung schneller korrekte Werte für die Konzentration des Sterilisationsmittels in der Behandlungsvorrichtung als im Stand der Technik angezeigt werden.

Die Aufgabe wird durch die Merkmale der unabhängigen Ansprüche gelöst. Vorteilhafte Weiterbildungen sind Gegenstand der abhängigen Ansprüche sowie der folgenden Beschreibung.

Dazu ist bei einer Behandlungsvorrichtung für Behälter und/oder Vorformlinge, aufweisend ein Gehäuse mit einem Innenraum und eine erste Messvorrichtung zum Messen einer Konzentration eines Fluids in einem ersten Konzentrationsbereich in dem Innenraum, erfindungsgemäß vorgesehen, dass die Behandlungsvorrichtung eine fluidkommunizierend mit dem Innenraum verbundene zweite Messvorrichtung zum Messen einer Konzentration des Fluids in einem zweiten Konzentrationsbereich in dem Innenraum und eine Trennvorrichtung für die erste Messvorrichtung aufweist, wobei die Trennvorrichtung in einem ersten Funktionszustand eine fluidkommunizierende Verbindung zwischen der ersten Messvorrichtung und dem Innenraum bereitstellt und in einem zweiten Funktionszustand die fluidkommunizierende Verbindung trennt, wobei der zweite Konzentrationsbereich mindestens einen Unterbereich aufweist, der höhere Konzentrationen als der erste Konzentrationsbereich aufweist.

Mit der Erfindung wird eine Behandlungsvorrichtung bereitgestellt, die zusätzlich zu der ersten Messvorrichtung eine zweite Messvorrichtung aufweist, die zumindest in den mindestens einen Unterbereich des zweiten Konzentrationsbereichs höhere Konzentrationen als die erste Messvorrichtung messen kann. Die erste Messvorrichtung kann daher für die Messung vergleichsweise niedriger Konzentrationen geeignet sein und die zweite Messvorrichtung für die Messung von vergleichsweise hohen Konzentrationen. Sobald die Konzentration ausgehend von einer niedrigen Konzentration weiter ansteigt, kann mit der Trennvorrichtung die Fluidverbindung zwischen der ersten Messvorrichtung und dem Innenraum der Behandlungsvorrichtung getrennt werden. Das heißt, dass die Trennvorrichtung dann in den zweiten Funktionszustand übergehen kann. Damit kann die erste Messrichtung vor hohen Konzentrationen des Fluids, die außerhalb des ersten Konzentrationsbereichs liegen, durch die Trennvorrichtung abgeschirmt werden. Wenn die Trennvorrichtung in dem zweiten Funktionszustand ist, wird die erste Messvorrichtung daher nicht mit hohen Konzentrationen des Fluids aus dem Innenraum beaufschlagt. Die Messung der Konzentration des Fluids kann dann mit der zweiten Messvorrichtung weitergeführt werden. Sobald die Konzentration absinkt, kann die Trennvorrichtung in den ersten Funktionszustand übergehen, um die Fluidverbindung zwischen dem Innenraum und der ersten Messvorrichtung wieder herzustellen. Die erste Messvorrichtung wird dann lediglich geringen Konzentration des Fluids ausgesetzt und kann daher deutlich schneller als im Stand der Technik korrekte Konzentrationswerte bereitstellen. Bedien-/Wartungspersonal kann daher früher in die Behandlungsvorrichtung als im Stand der Technik eintreten.

Der erste Konzentrationsbereich und der zweite Konzentrationsbereich können z. B. in einem Überlappungsbereich überlappen.

Damit kann ein kontinuierlicher Konzentrationsbereich durch die beiden Messvorrichtungen erfasst werden. Die Konzentration kann beim Wechsel zwischen den Konzentrationsbereichen lückenlos verfolgt werden.

Einem Beispiel gemäß kann die Trennvorrichtung ausgebildet sein, in den zweiten Funktionszustand zu wechseln, wenn die erste Messvorrichtung einen Messwert ausgibt, der größer als ein vordefinierter erster Schwellenwert ist, und in den ersten Funktionszustand zu wechseln, wenn die zweite Messvorrichtung einen Messwert ausgibt, der unterhalb eines zweiten vordefinierten Schwellenwerts ist.

Der erste Schwellenwert kann ein Konzentrationswert sein, der im ersten Konzentrationsbereichen angeordnet ist. Das Überschreiten des ersten Schwellenwerts nach oben, d. h. von geringeren Konzentrationen hin zu höheren Konzentrationen, kann den Wechsel der Trennvorrichtung in den zweiten Funktionszustand auslösen. D. h., dass die Fluidverbindung zwischen dem Innenraum und der ersten Messvorrichtung getrennt wird, sodass die erste Messvorrichtung keinen hohen Konzentrationen des Fluids ausgesetzt werden kann. Weiter kann das Unterschreiten des zweiten Schwellenwerts, der ein Konzentrationswert sein kann, der im zweiten Konzentrationsbereich angeordnet ist, von hohen Konzentrationen zu geringeren Konzentrationen dann von der zweiten Messvorrichtung erfasst werden. Sobald dies geschieht, kann die Trennvorrichtung in den ersten Funktionszustand gewechselt werden, sodass die erste Messvorrichtung wieder eine Fluidverbindung zu dem Innenraum aufweist.

Insbesondere, wenn ein Überlappungsbereich vorhanden ist, kann weiter vorgesehen sein, dass der erste und zweite Schwellenwert gleich sind und einen Konzentrationswert aufweisen, der im Überlappungsbereich angeordnet ist. D. h., sowohl die erste Messvorrichtung als auch die zweite Messvorrichtung können erfassen, ob ein Konzentrationswert den Schwellenwert nach oben oder nach unten überschreitet.

Einem Beispiel gemäß kann die Trennvorrichtung eine Fluidblockiereinrichtung, insbesondere ein Ventil, eine Klappe, eine Tür oder einen Schieber zwischen der ersten Messvorrichtung und dem Innenraum aufweisen.

Damit kann die erste Messvorrichtung durch das Schließen des Ventils oder des Schiebers fluidisch von dem Innenraum getrennt werden. Das Öffnen des Ventils oder des Schiebers kann eine Fluidverbindung zwischen dem Innenraum und der ersten Messvorrichtung herstellen.

Gemäß einem weiteren Beispiel kann die zweite Messvorrichtung in dem Innenraum angeordnet sein.

Die zweite Messvorrichtung kann damit unmittelbar die Konzentration in der Raumluft des Innenraums der Behandlungsvorrichtung erfassen. Eine Trennung der Fluidverbindung zwischen der zweiten Messvorrichtung und den Innenraum ist in diesem Fall nicht erforderlich. Die Messvorrichtung kann dann zum Beispiel kontinuierlich Messwerte für die Konzentration des Fluids in dem Innenraum erfassen, unabhängig davon, ob die erste Messvorrichtung durch die Trennvorrichtung von dem Innenraum getrennt wird. Damit wird die Handhabung der zweiten Messvorrichtung vereinfacht.

Weiter kann die Behandlungsvorrichtung zum Beispiel eine Steuervorrichtung zum Steuern der Trennvorrichtung und zum Empfangen von Messwerten von der ersten Messvorrichtung und der zweiten Messvorrichtung aufweisen.

Mit der Steuervorrichtung kann dann auf Basis der Messwerte der ersten Messvorrichtung bzw. der zweiten Vorrichtung der Wechsel der Trennvorrichtung zwischen dem ersten Funktionszustand und dem zweiten Funktionszustand gesteuert werden.

Einem anderen Beispiel gemäß kann eine Fluidleitung den Innenraum mit der ersten Messvorrichtung verbinden und die Trennvorrichtung die Fluidleitung im ersten Funktionszustand öffnen und im zweiten Funktionszustand schließen.

Die erste Messvorrichtung ist damit außerhalb des Innenraums des Gehäuses der Behälterstiftung angeordnet. Die Luft aus dem Innenraum des Gehäuses kann dann über die geöffnete Fluidleitung zu der ersten Messvorrichtung strömen, sodass die erste Messvorrichtung die Konzentration des Fluids in der Luft erfassen kann. Zum Beenden der Fluidverbindung zwischen dem Innenraum und der ersten Messvorrichtung kann die Fluidleitung durch die Trennvorrichtung geschlossen werden. Da die Fluidleitung einen, insbesondere im Vergleich zum Innenraum, kleinen Querschnitt aufweisen kann, kann die Baugröße der Trennvorrichtung kleingehalten werden.

Die Behandlungsvorrichtung kann beispielsweise weiter eine Spülvorrichtung zum Spülen der ersten Messvorrichtung mit einem Spülmedium aufweisen.

Die Spülvorrichtung kann die erste Messvorrichtung mit dem Spülmedium spülen, wenn die Trennvorrichtung im zweiten Funktionszustand ist. Fluid, das nach dem Trennen der Fluidverbindung zwischen den Innenraum und der ersten Messvorrichtung an der ersten Messvorrichtung angeordnet ist, kann durch das Spülen von der ersten Messvorrichtung zumindest weitestgehend entfernt werden. Wenn die erste Messvorrichtung über eine Fluidleitung mit dem Innenraum verbunden ist, kann das Fluid aus der Fluidleitung herausgespült werden.

Einem Beispiel gemäß kann die Spülvorrichtung ein Ventil und eine Spülleitung, die mittels des Ventils fluidkommunizierend mit der ersten Messvorrichtung verbindbar ist, aufweisen.

Die Spülleitung kann zum Beispiel an der oben erläuterten optionalen Fluidleitung angeschlossen sein. Der Anschluss der Spülleitung kann dabei zwischen der Trennvorrichtung und der ersten Messvorrichtung erfolgen. Die Spülleitung kann zum Beispiel mit einer Umgebung der Behandlungsvorrichtung fluidkommunizierend verbunden sein, wobei in dieser Umgebung keine oder lediglich Spuren von Fluid vorhanden sind. Maximal kann eine Konzentration von Fluid in der Umgebungsluft vorhanden sein, die kleiner als die oben genannten Grenzwerte sind.

Die Behandlungsvorrichtung kann zum Beispiel eine Absaugeinrichtung aufweisen, die fluidkommunizierend mit der ersten Messvorrichtung verbunden ist, wobei die erste Messvorrichtung vorzugsweise zwischen der Trennvorrichtung und der Absaugeinrichtung angeordnet ist.

Die Absaugeinrichtung kann zum Beispiel mit Fluid beaufschlagte Luft aus dem Innenraum zu der ersten Messvorrichtung saugen, wenn die Trennvorrichtung im ersten Funktionszustand ist. Wenn die Trennvorrichtung im zweiten Funktionszustand ist und eine Spülleitung vorgesehen ist, kann die Absaugeinrichtung beispielsweise Spülmedium, insbesondere Raumluft, die kein Fluid aufweist, zu der ersten Messvorrichtung saugen. Dazu kann die erste Messvorrichtung zwischen der Absaugeinrichtung und der Trennvorrichtung bzw. der Spülleitung angeordnet sein.

Einem Beispiel gemäß kann das Fluid ein Sterilisationsmittel, insbesondere Wasserstoffperoxid, sein.

Damit sei jedoch nicht ausgeschlossen, dass das Fluid alternativ oder zusätzlich ein anderes Sterilisationsmittel aufweisen kann, beispielsweise Peressigsäure, oder für einen anderen Zweck geeignet sein kann.

Einem weiteren Beispiel gemäß kann der zweite Konzentrationsbereich Konzentrationswerte zwischen 10 ppm bis 100000 ppm, vorzugweise 50 ppm bis 50000 ppm, weiter vorzugsweise 70 ppm bis 10000 ppm, am meisten bevorzugt von 100 ppm bis 7000 ppm, aufweisen.

Am Beispiel von Wasserstoffperoxid kann die Konzentration von Wasserstoffperoxid bei der Sterilisation einer Behandlungsvorrichtung im Bereich von 500 ppm bis 5000 ppm liegen. Daher kann die zweite Messvorrichtung mit dem oben genannten Konzentrationsbereich Werte für die Konzentrationen erfassen, die während der Sterilisation auftreten können und nicht von der ersten Messvorrichtung angezeigt werden können.

Weiter kann die Behandlungsvorrichtung zumindest eine Umformmaschine zum Umformen von Vorformlingen zu Behältern und/oder eine Füllmaschine zum Füllen von Behältern aufweisen. Das Umformen kann beispielsweise durch Streckblasen oder durch das Füllen mit dem späteren Inhalt des Behälters erfolgen.

Weiter betrifft die Erfindung ein Verfahren zum Steuern einer Behandlungsvorrichtung nach der vorangegangenen Beschreibung, wobei die Trennvorrichtung in den zweiten Funktionszustand wechselt, wenn die erste Messvorrichtung einen Messwert für die Konzentration ausgibt, der größer als ein erster vordefinierter Schwellenwert, und in den ersten Funktionszustand wechseln, wenn die zweite Messvorrichtung einen Messwert für die Konzentration ausgibt, der unterhalb eines zweiten vordefinierten Schwellenwerts ist.

Gemäß einem Beispiel kann eine Steuervorrichtung der Behandlungsvorrichtung zum Steuern der Behandlungsvorrichtung, insbesondere für Türzuhaltungen, eine Absaugung, eine Belüftung, einen Produktionsstopp und/oder eine Produktionsfreigabe, Messwerte der ersten Messvorrichtung berücksichtigen, wenn die Trennvorrichtung im ersten Funktionszustand ist, und Messwerte der zweiten Messvorrichtung berücksichtigen, wenn die Trennvorrichtung im zweiten Funktionszustand ist.

Vorteile und Wirkungen sowie Weiterbildungen des Verfahrens ergeben sich aus den Vorteilen und Wirkungen sowie Weiterbildungen der oben beschriebenen Behandlungsvorrichtung. Zur Vermeidung von Wiederholungen wird daher in dieser Hinsicht auf die vorangegangene Beschreibung verwiesen.

Im Folgenden wird die Erfindung anhand von beispielhaften Ausführungsformen mittels der beigefügten Zeichnung beschrieben. Es zeigen:
- Figur 1: eine schematische Darstellung der Behandlungsvorrichtung gemäß einem ersten Ausführungsbeispiel;
- Figur 2: eine schematische Darstellung der Behandlungsvorrichtung gemäß einem zweiten Ausführungsbeispiel;
- Figur 3: eine schematische Darstellung der Behandlungsvorrichtung gemäß einem dritten Ausführungsbeispiel;
- Figur 4: eine schematische Darstellung von Konzentrationsbereichen und Überlappungsbereichen; und
- Figur 5: ein Flussdiagramm des Verfahrens.

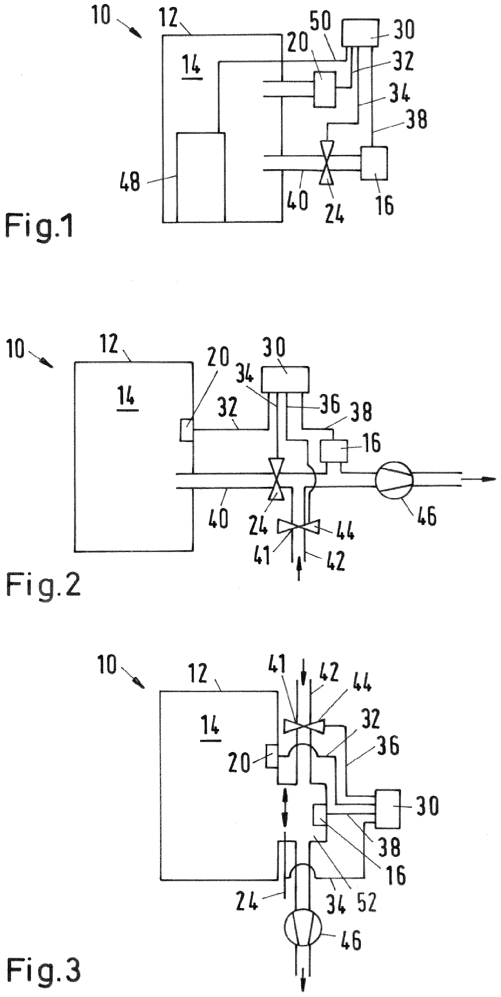

Die Behandlungsvorrichtung wird im Folgenden in ihrer Gesamtheit mit dem Bezugszeichen 10 bezeichnet, wie beispielsweise in Figur 1 dargestellt.

Die Behandlungsvorrichtung 10 ist zum Behandeln von Behältern und/oder Vorformlingen ausgebildet. Unter einer Behandlung von Behältern und/oder Vorformlingen wird zum Beispiel das Umformen von Vorformlingen zu Behältern oder das Füllen von Behältern verstanden. Weiter Behandlungsmöglichkeiten seien damit jedoch nicht ausgeschlossen.

Die Behandlungsvorrichtung 10 weist ein Gehäuse 12 auf, das einen Innenraum 14 umschließt. Das Gehäuse 12 kann ein Türelement 48 für Bedienpersonal aufweisen, durch das Bedienpersonal in den Innenraum 14 der Behandlungsvorrichtung 10 eintreten kann.

Weiter ist eine erste Messvorrichtung 16 fluidisch mit dem Innenraum 14 verbunden. Dazu kann die erste Messvorrichtung 16 über eine Fluidleitung 40 mit dem Innenraum 14 verbunden sein. Die Fluidleitung 40 kann durch eine Trennvorrichtung 24 beherrscht werden. Dazu ist die Fluidleitung 40 in einem ersten Funktionszustand der Trennvorrichtung 24 geöffnet, sodass eine Fluidverbindung zwischen dem Innenraum 14 und der ersten Messvorrichtung 16 besteht. In einem zweiten Funktionszustand der Trennvorrichtung 24 ist die Fluidleitung 40 gesperrt, sodass keine Fluidverbindung zwischen dem Innenraum 14 und der ersten Messvorrichtung 16 mehr besteht.

In diesem Beispiel ist die Trennvorrichtung 24 als Ventil ausgebildet, mit dem die Fluidleitung 40 geöffnet und geschlossen werden kann.

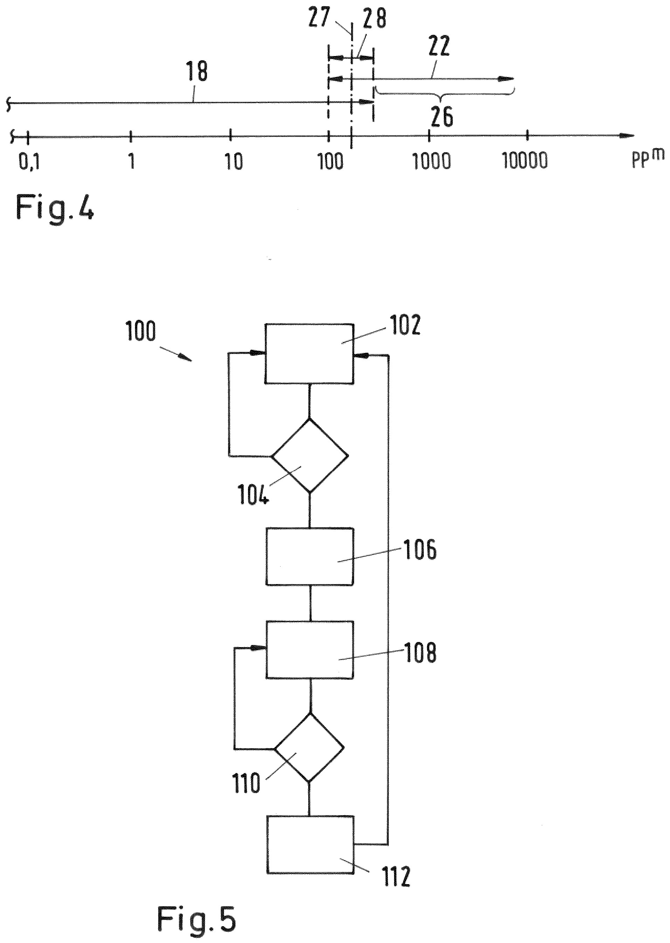

Die erste Messvorrichtung 16 weist einen ersten Messbereich 18 für Konzentrationen eines Fluids, insbesondere Wasserstoffperoxid in Luft, auf, wie in Figur 4 dargestellt ist. Der erste Messbereich 18 umfasst dabei Konzentrationswerte, die unterhalb eines gesetzlichen Grenzwerts für das Fluid in der Luft liegen, bei denen das Bedienpersonal den Innenraum 14 betreten kann. Dieser kann zum Beispiel bei 0,5 ppm liegen. D. h., dass der erste Messbereich 18 sich zumindest um den Konzentrationswert von 0,5 ppm erstreckt.

Die Behandlungsvorrichtung 10 weist weiter eine zweite Messvorrichtung 20 auf, die fluidisch mit dem Innenraum 14 verbunden ist. Die zweite Messvorrichtung 20 weist einen zweiten Messbereich 22 auf. Der zweite Messbereich 22 überlappt gemäß Figur 4 mit dem ersten Messbereich 18. Weiter weist der zweite Messbereich 22 einen Unterbereich 26 auf, der höhere Konzentrationswerte als der erste Messbereich 18 aufweist. Der zweite Messbereich 22 ist daher im Vergleich zum ersten Messbereich 22 zu höheren Konzentrationen verschoben.

Die Trennvorrichtung 24 kann dazu ausgebildet sein, in den zweiten Funktionszustand überzugehen, wenn die erste Messvorrichtung 16 Messwerte ermittelt, die im Überlappungsbereich 28 sind und weiter ansteigen. Dazu kann zum Beispiel ein Schwellenwert 27 in den Überlappungsbereich 28 definiert sein. Sobald die erste Messvorrichtung 16 eine Konzentration misst, die größer als der Schwellenwert 27 ist, kann die Trennvorrichtung 24 in den zweiten Funktionszustand übergehen.

Weiter ist denkbar, dass in einem weiteren Beispiel zwei Schwellenwerte definiert werden können. So kann beispielsweise bei steigenden Werten ein niedriger erster Schwellenwert verwendet werden, um die erste Messvorrichtung 16 rechtzeitig zu sperren, damit sie bei schnell steigenden Werten nicht überlastet wird. Bei sinkenden Werten kann dann beispielsweise ein hoher zweiter Schwellenwert verwendet werden, so dass die erste Messvorrichtung 16 früher wieder angeschaltet werden kann, wenn durch die zweite Messvorrichtung 20 ermittelt wird, dass die Konzentration unterhalb des zweiten Schwellenwerts gesunken ist.

Durch das Trennen der Fluidverbindung zwischen dem Innenraum 14 und der ersten Messvorrichtung 16 wird die erster Messvorrichtung 16 von dem Innenraum 14 abgekoppelt. Die erste Messvorrichtung 16 kann damit als abgeschaltet angesehen werden.

Umgekehrt kann die Trennvorrichtung 24 weiter dazu ausgebildet sein, in den ersten Funktionszustand überzugehen, wenn die zweite Messvorrichtung 20 Messwerte ermittelt, die im Überlappungsbereich 28 sind und weiter fallen. So kann zum Beispiel auch hier der Schwellenwert 27 herangezogen werden. Sobald die zweite Messvorrichtung 20 Messwerte ermittelt, die niedriger als der Schwellenwert 27 sind, kann die Trennvorrichtung 24 in den ersten Funktionszustand übergehen.

Die zweite Messvorrichtung 20 kann ausgebildet sein, kontinuierlich Messwerte bereitzustellen. Eine Trennung der Fluidverbindung zwischen der zweiten Messvorrichtung 20 und dem Innenraum 14 beim Unterschreiten des Schwellenwerts 27 ist nicht notwendig.

Die Behandlungsvorrichtung 10 kann weiter eine Steuervorrichtung 30 aufweisen, die ausgebildet sein kann, die von der ersten Messvorrichtung 16 oder der zweiten Messvorrichtung 20 bereitgestellten Messwerte auszuwerten und mit dem Schwellenwert 27 zu vergleichen. Dazu kann die Steuervorrichtung 30 über eine erste Signalverbindung 38 mit der ersten Messvorrichtung 16 verbunden sein. Über eine zweite Signalverbindung 32 kann die Steuervorrichtung 30 mit der zweiten Messvorrichtung 20 verbunden sein.

Über eine dritte Signalverbindung 34 kann die Steuervorrichtung 30 mit der Trennvorrichtung 24 verbunden sein. Die Signalverbindungen können kabellos oder kabelgebunden ausgebildet sein.

Solange die Messwerte für die Konzentration des Fluids unterhalb des Schwellenwerts 27 sind, kann die Steuervorrichtung 30 lediglich Messwerte der ersten Messvorrichtung 16 berücksichtigen. Sofern die Messwerte der ersten Messvorrichtung 16 anzeigen, dass die Konzentration des Fluids den Schwellenwert 27 überschreitet, kann die Steuervorrichtung 30 ein Signal über die dritte Signalverbindung 34 an die Trennvorrichtung 24 ausgeben, mit dem die Trennvorrichtung 24 in den zweiten Funktionszustand geschaltet wird. Damit wird die Fluidverbindung zwischen der ersten Messvorrichtung 16 und den Innenraum 14 geschlossen.

Ab diesem Zeitpunkt kann die Steuervorrichtung 30 Messwerte der zweiten Messvorrichtung 20 verwenden, um die Konzentration des Fluids im Innenraum 14 zu überwachen. Messwerte der ersten Messvorrichtung 16 können dann unberücksichtigt bleiben.

Sobald die Messwerte der zweiten Messvorrichtung 20 anzeigen, dass die Konzentration des Fluid den Schwellenwert 27 wieder unterschreitet, kann die Steuervorrichtung 30 ein weiteres Signal an die Trennvorrichtung 24 ausgeben, mit dem die Trennvorrichtung 24 in den ersten Funktionszustand geschaltet wird. Die Fluidverbindung zwischen der ersten Messvorrichtung 16 und dem Innenraum 14 wird dann wieder geöffnet.

Sobald die Fluidverbindung wieder geöffnet ist, kann die Steuervorrichtung 30 Messwerte der ersten Messvorrichtung 16 für die Überwachung der Konzentration des Fluids verwenden. Messwerte der zweiten Messvorrichtung 20 können dann unberücksichtigt bleiben.

Weiter kann zum Beispiel das Türelement 48 über eine Steuersignal, das über eine vierte Signalverbindung 50 an das Türelement 48 übermittelt werden kann, bereits dann verriegelt werden, wenn der gesetzliche Grenzwert für die Konzentration des Fluids in dem Innenraum 14 überschritten wird.

Weiter kann die Steuervorrichtung 30 bei Unterschreiten des gesetzlich festgelegten Grenzwerts der Konzentration ein Steuersignal an das Türelement 48 übermitteln, mit dem das Türelement 48 wieder entriegelt wird. Der Zugang zu dem Innenraum 14 wird für Bedienpersonal damit wieder ermöglicht.

In Figur 2 wird ein weiteres Ausführungsbeispiel der Behandlungsvorrichtung 10 dargestellt. Im Vergleich zum Beispiel nach Figur 1 ist die zweite Messvorrichtung 20 unmittelbar in dem Innenraum 14 angeordnet. Dazu kann die zweite Messvorrichtung 20 zum Beispiel an dem Gehäuse 12 befestigt sein.

In diesem Ausführungsbeispiel nach Figur 2 kann die Behandlungsvorrichtung 10 eine Absaugeinrichtung 46 aufweisen. Die Absaugeinrichtung 46 kann mit der ersten Messvorrichtung 16 fluidverbunden sein. Die Absaugeinrichtung 46 bewirkt bei ihrem Betrieb, dass Luft von der ersten Messvorrichtung 16 abgesaugt wird. Wenn die Trennvorrichtung 24 im ersten Funktionszustand ist, saugt die Absaugeinrichtung 46 daher Luft aus dem Innenraum 14 zu der ersten Messvorrichtung 16. Nach dem Wechsel der Trennvorrichtung 24 in den ersten Funktionszustand kann damit die korrekte Konzentration des Fluids in der Luft im Innenraum 14 noch schneller durch die erste Messvorrichtung 16 ermittelt werden. Sobald die Luft die erste Messvorrichtung 16 und die Absaugeinrichtung 46 passiert hat, kann die Luft über eine Abgasleitung in die Umgebung ausgelassen werden.

Die Absaugeinrichtung 46 kann derart angeordnet sein, dass die erste Messvorrichtung 16 zwischen der Absaugeinrichtung 46 und dem Innenraum 14 angeordnet ist.

Weiter kann die Behandlungsvorrichtung 10 in diesem Ausführungsbeispiel eine Spülvorrichtung 41 aufweisen. Die Spülvorrichtung 41 kann eine Spülleitung 42 aufweisen, die mit der ersten Messvorrichtung 16 fluidverbunden ist. Da die erste Messvorrichtung 16 in diesem Beispiel über eine Fluidleitung 40 mit dem Innenraum 14 verbunden ist, kann die Spülleitung 42 von der Fluidleitung 40 abzweigen. Die Spülleitung 42 ist dabei zwischen der Trennvorrichtung 24 und der ersten Messvorrichtung 16 mit der ersten Messvorrichtung 16 fluidverbunden.

Weiter weist die Spülvorrichtung 41 ein Ventil 44 auf, das die Spülleitung 42 beherrscht. Über das Öffnen des Ventils 44 wird die Spülleitung 42 freigegeben. Wenn das Ventil 44 geschlossen ist, ist die Spülleitung 42 gesperrt.

Die Steuervorrichtung 30 kann dazu über einer fünfte Signalverbindung 36 mit dem Ventil 44 verbunden sein. Über die fünfte Signalverbindung 36 kann die Steuervorrichtung 30 Steuersignale an das Ventil 44 übermitteln, um das Ventil 44 zu Öffnen oder zu Schließen.

Wenn die Trennvorrichtung 24 in den ersten Funktionszustand übergeht, kann die Steuervorrichtung 30 das Ventil 44 anweisen zu schließen. Wenn die Trennvorrichtung 24 in den zweiten Funktionszustand übergeht, kann die Steuervorrichtung 30 das Öffnen des Ventils 44 anweisen. Damit ist die Spülleitung 42 nur dann geöffnet, wenn die Fluidverbindung zwischen der ersten Messvorrichtung 16 dem Innenraum 14 nicht besteht.

Wenn die Trennvorrichtung 24 im zweiten Funktionszustand ist, kann die Absaugeinrichtung 46 über die Spülleitung 42 bei geöffnetem Ventil 44 Spülmedium, zum Beispiel Luft aus der Umgebung, zu der ersten Messvorrichtung 16 fördern. Mit dem Spülmedium kann Luft, die mit dem Fluid, das zum Beispiel Sterilisationsmittel sein kann, aus der ersten Messvorrichtung 16 gespült werden.

Gemäß Figur 3 wird ein drittes Ausführungsbeispiel der Behandlungsvorrichtung 10 dargestellt. In diesem Beispiel ist die erste Messvorrichtung 16 in einem von dem Innenraum 14 abtrennbaren Bereich 52 des Gehäuses 12 angeordnet. Der abtrennbare Bereich 52 kann durch die Trennvorrichtung 24 von dem Rest des Innenraums 14 abgetrennt werden. Die Trennvorrichtung 24 kann dazu zum Beispiel als Schieber ausgebildet sein.

Die Spülleitung 42 kann in diesem Beispiel von dem abtrennbaren Bereich 52 abzweigen. Weiter kann die Absaugeinrichtung 46 an den abtrennbaren Bereich 52 angeschlossen sein.

Figur 4 stellt die Konzentrationsbereiche 18, 22 der ersten Messvorrichtung 16 und der zweiten Messrichtung 20 anhand einer logarithmischen dargestellten Skala dar. Die Skala zeigt dabei von links nach rechts aufsteigend die Konzentration des Fluids in der Luft.

Der erste Konzentrationsbereich 18 der ersten Messvorrichtung 16 kann sich beispielsweise von weniger als 0,1 ppm bis ca. 300 ppm erstrecken. Andere Grenzwerte des ersten Konzentrationsbereichs 18 seien damit jedoch nicht ausgeschlossen.

Der zweite Konzentrationsbereich 22 kann sich zum Beispiel von einem Konzentrationswert von 100 ppm bis ca. 7000 ppm erstrecken. Auch hier sind andere Grenzwerte für den zweiten Konzentrationsbereich 22 denkbar.

Der zweite Konzentrationsbereich 22 umfasst mindestens einen Unterbereich 26, der höhere Konzentrationswerte als der erste Konzentrationsbereich 18 aufweist.

In dem Überlappungsbereich 28 überlappen der erste Konzentrationsbereich 18 und der zweite Konzentrationsbereich 22. Der Überlappungsbereich 28 kann sich in diesem Beispiel von 100 ppm bis 300 ppm erstrecken. Im Überlappungsbereich 28 können sowohl die erste Messvorrichtung 16 als auch die die zweite Messvorrichtung 20 korrekte Messwerte liefern.

Im Überlappungsbereich 28 kann ein Schwellenwert 27 definiert sein, dessen Überschreiten einen Wechsel des Funktionszustands der Trennvorrichtung 24 auslöst.

Dies ist beispielsweise mit dem Flussdiagramm aus Figur 5 dargestellt, das eine Ausführungsform des Verfahrens 100 darstellt.

Ausgehend von einer Situation, in der die Trennvorrichtung 24 im ersten Funktionszustand ist und die Messvorrichtung 16 Messwerte anzeigt, die eine Konzentration von Fluid im Bereich unterhalb von 0,5 ppm anzeigen, kann in einem ersten Schritt 102 zum Beispiel durch die Steuervorrichtung 30 eine Erfassung der Messwerte der ersten Messvorrichtung 16 erfolgen.

In einem weiteren Schritt 104 kann, zum Beispiel durch die Steuervorrichtung 30, verglichen werden, ob die Messwerte den Schwellenwert 27 überschreiten. Ist dies nicht der Fall, erfolgt eine weitere Erfassung der Messwerte der ersten Messvorrichtung 16 gemäß Schritt 102.

Überschreiten die Messwerte den Schwellenwert 27, wird mit dem Schritt 106 fortgefahren. In diesem Schritt wird die Trennvorrichtung 24 in den zweiten Funktionszustand überführt. Weiter werden nunmehr die Messwerte der zweiten Messvorrichtung 20 verwendet und nicht mehr die der ersten Messvorrichtung zwei 16.

Im Schritt 108 werden daher die Messwerte der zweiten Messvorrichtung 20, zum Beispiel durch die Steuervorrichtung 30, erfasst.

In einem weiteren Schritt 110 kann ein Vergleich durchgeführt werden, ob die Messwerte der zweiten Messvorrichtung 20 den Schwellenwert 27 unterschreiten. Dies kann zum Beispiel durch die Steuervorrichtung 30 durchgeführt werden.

Ist dies nicht der Fall, wird mit Schritt 108 fortgefahren und es werden weiter die Messwerte der zweiten Messvorrichtung 20 verwendet.

Unterschreiten die Messwerte der zweiten Messvorrichtung 20 den Schwellenwert 27, wird die Trennvorrichtung 24 gemäß Schritt 112 in den ersten Funktionszustand geführt. Weiter werden von nun an die Messwerte der ersten Messvorrichtung 16 verwendet und die der zweiten Messvorrichtung 20 nicht weiter berücksichtigt.

Im Weiteren kann dann mit dem Schritt 102 fortgefahren werden, bei dem die Messwerte der ersten Messvorrichtung 16 erfasst werden.

Optional kann das Verfahren 100 weitere Schritte aufweisen, zum Beispiel eine Freischaltung bzw. Entriegelung des Türelements 48, wenn die Konzentration beispielsweise unterhalb von 0,5 ppm liegt.

Alle oben genannten Schritte können durch die Steuervorrichtung 30 durchgeführt werden.

### Bezugszeichenliste

- 10: Behandlungsvorrichtung
- 12: Gehäuse
- 14: Innenraum
- 16: erste Messvorrichtung
- 18: erster Konzentrationsbereich
- 20: zweite Messvorrichtung
- 22: zweiter Konzentrationsbereich
- 24: Trennvorrichtung
- 26: Unterbereich
- 27: Schwellenwert
- 28: Überlappungsbereich
- 30: Steuervorrichtung
- 32: zweite Signalverbindung
- 34: dritte Signalverbindung
- 36: fünfte Signalverbindung
- 38: erste Signalverbindung
- 40: Fluidleitung
- 41: Spülvorrichtung
- 42: Spülleitung
- 44: Ventil
- 46: Absaugeinrichtung
- 48: Türelement
- 50: vierte Signalverbindung
- 52: abtrennbarer Bereich

## Patentansprüche

1. Behandlungsvorrichtung (10) für Behälter und/oder Vorformlinge, umfassend ein Gehäuse (12) mit einem Innenraum (14) und eine erste Messvorrichtung (16) zum Messen einer Konzentration eines Fluids in einem ersten Konzentrationsbereich (18) in dem Innenraum (14), wobei die Behandlungsvorrichtung (10) eine fluidkommunizierend mit dem Innenraum (14) verbundene zweite Messvorrichtung (20) zum Messen einer Konzentration des Fluids in einem zweiten Konzentrationsbereich (22) in dem Innenraum (14) aufweist, **dadurch gekennzeichnet, dass** die Behandlungsvorrichtung (10) eine Trennvorrichtung (24) für die erste Messvorrichtung (16) aufweist, wobei die Trennvorrichtung (24) in einem ersten Funktionszustand eine fluidkommunizierende Verbindung zwischen der ersten Messvorrichtung (16) und dem Innenraum (14) bereitstellt und in einem zweiten Funktionszustand die fluidkommunizierende Verbindung trennt, wobei der zweite Konzentrationsbereich (22) mindestens einen Unterbereich (26) aufweist, der höhere Konzentrationen als der erste Konzentrationsbereich (18) aufweist.

2. Behandlungsvorrichtung (10) nach Anspruch 1, **dadurch gekennzeichnet, dass** der erste und zweite Konzentrationsbereich (22) in einem Überlappungsbereich (28) überlappen.

3. Behandlungsvorrichtung (10) nach Anspruch 1 oder 2, **dadurch gekennzeichnet, dass** die Trennvorrichtung (24) ausgebildet ist, in den zweiten Funktionszustand zu wechseln, wenn die erste Messvorrichtung (16) einen Messwert ausgibt, der größer als ein erster vordefinierter Schwellenwert (27) ist, und in den ersten Funktionszustand zu wechseln, wenn die zweite Messvorrichtung (20) einen Messwert ausgibt, der unterhalb eines zweiten vordefinierten Schwellenwerts (27) ist.

4. Behandlungsvorrichtung (10) nach einem der vorangegangenen Ansprüche, **dadurch gekennzeichnet, dass** die Trennvorrichtung (24) eine Fluidblockiereinrichtung, insbesondere ein Ventil, eine Klappe, eine Tür oder einen Schieber, zwischen der ersten Messvorrichtung (16) und dem Innenraum (14) aufweist.

5. Behandlungsvorrichtung (10) nach einem der vorangegangenen Ansprüche, **dadurch gekennzeichnet, dass** die zweite Messvorrichtung (20) in dem Innenraum (14) angeordnet ist.

6. Behandlungsvorrichtung (10) nach einem der vorangegangenen Ansprüche, **dadurch gekennzeichnet, dass** die Behandlungsvorrichtung (10) weiter eine Steuervorrichtung (30) zum Steuern der Trennvorrichtung (24) und zum Empfangen von Messwerten von der ersten Messvorrichtung (16) und der zweiten Messvorrichtung (20) aufweist.

7. Behandlungsvorrichtung (10) nach einem der vorangegangenen Ansprüche, **dadurch gekennzeichnet, dass** eine Fluidleitung (40) den Innenraum (14) mit der ersten Messvorrichtung (16) verbindet und die Trennvorrichtung (24) die Fluidleitung (40) im ersten Funktionszustand öffnet und im zweiten Funktionszustand schließt.

8. Behandlungsvorrichtung (10) nach einem der vorangegangenen Ansprüche, **dadurch gekennzeichnet, dass** die Behandlungsvorrichtung (10) weiter eine Spülvorrichtung (41) zum Spülen der ersten Messvorrichtung (16) mit einem Spülmedium aufweist.

9. Behandlungsvorrichtung (10) nach dem vorangegangenen Anspruch, **dadurch gekennzeichnet, dass** die Spülvorrichtung (41) ein Ventil (44) und eine Spülleitung (42), die mittels des Ventils fluidkommunizierend mit der ersten Messvorrichtung (16) verbindbar ist, aufweist.

10. Behandlungsvorrichtung (10) nach einem der vorangegangenen Ansprüche,
**dadurch gekennzeichnet, dass** die Behandlungsvorrichtung (10) eine Absaugeinrichtung (46) aufweist, die fluidkommunizierend mit der ersten Messvorrichtung (16) verbunden ist, wobei die erste Messvorrichtung (16) vorzugsweise zwischen der Trennvorrichtung (24) und der Absaugeinrichtung (46) angeordnet ist.

11. Behandlungsvorrichtung (10) nach einem der vorangegangenen Ansprüche,
**dadurch gekennzeichnet, dass** das Fluid ein Sterilisationsmittel, insbesondere Wasserstoffperoxid, ist.

12. Behandlungsvorrichtung (10) nach einen der vorangegangenen Ansprüche,
**dadurch gekennzeichnet, dass** der zweite Konzentrationsbereich (22) Konzentrationswerte zwischen 10 ppm bis 100000 ppm, vorzugweise 50 ppm bis 50000 ppm, weiter vorzugsweise 70 ppm bis 10000 ppm, am meisten bevorzugt von 100 ppm bis 7000 ppm, aufweist.

13. Behandlungsvorrichtung (10) nach einem der vorangegangenen Ansprüche,
**dadurch gekennzeichnet, dass** die Behandlungsvorrichtung (10) zumindest eine Umformmaschine zum Umformen von Vorformlingen zu Behältern und/oder eine Füllmaschine zum Füllen von Behältern aufweist.

14. Verfahren (100) zum Steuern einer Behandlungsvorrichtung (10) nach einem der vorangegangenen Ansprüche, wobei die Trennvorrichtung (24) in den zweiten Funktionszustand wechselt (106), wenn die erste Messvorrichtung (16) einen Messwert für die Konzentration ausgibt, der größer als ein erster vordefinierter Schwellenwert (27) ist, und in den ersten Funktionszustand wechselt (112), wenn die zweite Messvorrichtung (20) einen Messwert für die Konzentration ausgibt, der unterhalb eines zweiten vordefinierten Schwellenwerts (27) ist.

15. Verfahren (100) nach Anspruch 14, **dadurch gekennzeichnet, dass** eine Steuervorrichtung (30) der Behandlungsvorrichtung (10) zum Steuern der Behandlungsvorrichtung (10), insbesondere für Türzuhaltungen, eine Absaugung, eine Belüftung, einen Produktionsstopp und/oder eine Produktionsfreigabe, Messwerte der ersten Messvorrichtung (16) berücksichtigt (102), wenn die Trennvorrichtung (24) im ersten Funktionszustand ist, und Messwerte der zweiten Messvorrichtung (20) berücksichtigt (108), wenn die Trennvorrichtung (24) im zweiten Funktionszustand ist.

## Claims

1. Treatment device (10) for containers and/or preforms, comprising a housing (12) with an interior space (14) and a first measuring device (16) for measuring a concentration of a fluid in a first concentration range (18) in the interior space (14), wherein the treatment device (10) has a second measuring device (20) connected to the interior space (14) in a fluid-communicating manner for measuring a concentration of the fluid in a second concentration range (22) in the interior space (14), **characterised in that** the treatment device (10) has a separating device (24) for the first measuring device (16), wherein the separating device (24) in a first functional state provides a fluid-communicating connection between the first measuring device (16) and the interior space (14) and in a second functional state separates the fluid-communicating connection, the second concentration range (22) having at least one subrange (26) which has higher concentrations than the first concentration range (18).

2. Treatment device (10) according to claim 1, **characterised in that** the first and second concentration ranges (22) overlap in an overlap range (28).

3. Treatment device (10) according to claim 1 or 2, **characterised in that** the separating device (24) is configured to switch to the second functional state when the first measuring device (16) outputs a measurement that is above a first predefined threshold value (27), and to switch to the first functional state when the second measuring device (20) outputs a measurement which is below a second predefined threshold value (27).

4. Treatment device (10) according to any of the preceding claims,
**characterised in that** the separating device (24) has a fluid blocking device, in particular a valve, a flap, a door or a slide, between the first measuring device (16) and the interior space (14).

5. Treatment device (10) according to any of the preceding claims,
**characterised in that** the second measuring device (20) is arranged in the interior space (14).

6. Treatment device (10) according to any of the preceding claims,
**characterised in that** the treatment device (10) further comprises a control device (30) for controlling the separating device (24) and for receiving measurements from the first measuring device (16) and the second measuring device (20).

7. Treatment device (10) according to any of the preceding claims,
**characterised in that** a fluid line (40) connects the interior space (14) to the first measuring device (16), and the separating device (24) opens the fluid line (40) in the first functional state and closes it in the second functional state.

8. Treatment device (10) according to any of the preceding claims,
**characterised in that** the treatment device (10) further comprises a rinsing device (41) for rinsing the first measuring device (16) with a rinsing medium.

9. Treatment device (10) according to the preceding claim,
**characterised in that** the rinsing device (41) has a valve (44) and a rinsing line (42) which can be connected to the first measuring device (16) in a fluid-communicating manner by means of the valve.

10. Treatment device (10) according to any of the preceding claims,
**characterised in that** the treatment device (10) has a suction device (46) which is connected to the first measuring device (16) in a fluid-communicating manner, the first measuring device (16) preferably being arranged between the separating device (24) and the suction device (46).

11. Treatment device (10) according to any of the preceding claims, **characterised in that** the fluid is a sterilising agent, in particular hydrogen peroxide.

12. Treatment device (10) according to any of the preceding claims,
**characterised in that** the second concentration range (22) has concentration values from 10 ppm to 100,000 ppm, preferably 50 ppm to 50,000 ppm, more preferably 70 ppm to 10,000 ppm, most preferably from 100 ppm to 7,000 ppm.

13. Treatment device (10) according to any of the preceding claims,
**characterised in that** the treatment device (10) has at least one forming machine for converting preforms into containers and/or a filling machine for filling containers.

14. Method (100) for controlling a treatment device (10) according to any of the preceding claims, wherein the separating device (24) switches to the second functional state (106) when the first measuring device (16) outputs a concentration measurement that is above a first predefined threshold value (27), and switches to the first functional state (112) when the second measuring device (20) outputs a concentration measurement which is below a second predefined threshold value (27).

15. Method (100) according to claim 14, **characterised in that** a control device (30) of the treatment device (10) for controlling the treatment device (10), in particular for door locking, suction, ventilation, production stoppage and/or production release, takes into account (102) measurements of the first measuring device (16) when the separating device (24) is in the first functional state, and takes into account (108) measurements of the second measuring device (20) when the separating device (24) is in the second functional state.

## Revendications

1. Dispositif de traitement (10) pour récipients et/ou préformes, comprenant un boîtier (12) avec un espace intérieur (14) et un premier dispositif de mesure (16) pour mesurer une concentration d'un fluide dans une première plage de concentration (18) dans l'espace intérieur (14), dans lequel le dispositif de traitement (10) comprend un deuxième dispositif de mesure (20) relié en communication fluidique à l'espace intérieur (14) pour mesurer une concentration du fluide dans une deuxième plage de concentration (22) dans l'espace intérieur (14), **caractérisé en ce que** le dispositif de traitement (10) présente un dispositif de séparation (24) pour le premier dispositif de mesure (16), dans lequel le dispositif de séparation (24) fournit, dans un premier état de fonctionnement, une communication fluidique entre le premier dispositif de mesure (16) et l'espace intérieur (14) et sépare, dans un deuxième état de fonctionnement, la communication fluidique, dans lequel la deuxième zone de concentration (22) présente au moins une sous-plage (26), qui présente des concentrations plus élevées que la première plage de concentration (18).

2. Dispositif de traitement (10) selon la revendication 1, **caractérisé en ce que** les première et deuxième plages de concentration (22) se chevauchent dans une plage de chevauchement (28).

3. Dispositif de traitement (10) selon la revendication 1 ou 2, **caractérisé en ce que** le dispositif de séparation (24) est réalisé pour passer dans le deuxième état de fonctionnement, lorsque le premier dispositif de mesure (16) émet une valeur de mesure qui est supérieure à une première valeur de seuil prédéfinie (27), et pour passer dans le premier état de fonctionnement lorsque le deuxième dispositif de mesure (20) émet une valeur de mesure, qui est inférieure à une deuxième valeur de seuil prédéfinie (27).

4. Dispositif de traitement (10) selon l'une quelconque des revendications précédentes, **caractérisé en ce que** le dispositif de séparation (24) présente un système de blocage de fluide, en particulier une vanne, un clapet, une porte ou un tiroir, entre le premier dispositif de mesure (16) et l'espace intérieur (14).

5. Dispositif de traitement (10) selon l'une quelconque des revendications précédentes, **caractérisé en ce que** le deuxième dispositif de mesure (20) est disposé dans l'espace intérieur (14).

6. Dispositif de traitement (10) selon l'une quelconque des revendications précédentes, **caractérisé en ce que** le dispositif de traitement (10) présente en outre un dispositif de commande (30) pour commander le dispositif de séparation (24) et pour recevoir des valeurs de mesure du premier dispositif de mesure (16) et du deuxième dispositif de mesure (20).

7. Dispositif de traitement (10) selon l'une quelconque des revendications précédentes, **caractérisé en ce qu'**une conduite de fluide (40) relie l'espace intérieur (14) au premier dispositif de mesure (16) et le dispositif de séparation (24) ouvre la conduite de fluide (40) dans le premier état de fonctionnement et la ferme dans le deuxième état de fonctionnement.

8. Dispositif de traitement (10) selon l'une quelconque des revendications précédentes, **caractérisé en ce que** le dispositif de traitement (10) présente par ailleurs un dispositif de rinçage (41) pour rincer le premier dispositif de mesure (16) avec un milieu de rinçage.

9. Dispositif de traitement (10) selon la revendication précédente, **caractérisé en ce que** le dispositif de rinçage (41) présente une vanne (44) et une conduite de rinçage (42) qui peut être reliée au premier dispositif de mesure (16) en communication fluidique au moyen de la vanne.

10. Dispositif de traitement (10) selon l'une quelconque des revendications précédentes, **caractérisé en ce que** le dispositif de traitement (10) présente un système d'évacuation par aspiration (46) qui est relié en communication fluidique au premier dispositif de mesure (16), dans lequel le premier dispositif de mesure (16) est disposé de préférence entre le dispositif de séparation (24) et le dispositif d'évacuation par aspiration (46).

11. Dispositif de traitement (10) selon l'une quelconque des revendications précédentes, **caractérisé en ce que** le fluide est un agent stérilisant, en particulier du peroxyde d'hydrogène.

12. Dispositif de traitement (10) selon l'une quelconque des revendications précédentes, **caractérisé en ce que** la deuxième plage de concentration (22) présente des valeurs de concentration comprises entre 10 ppm et 100 000 ppm, de préférence entre 50 ppm et 50 000 ppm, en outre de préférence entre 70 ppm et 10 000 ppm, idéalement entre 100 ppm et 7 000 ppm.

13. Dispositif de traitement (10) selon l'une quelconque des revendications précédentes, **caractérisé en ce que** le dispositif de traitement (10) présente au moins une machine de mise en forme pour la mise en forme de préformes en contenants et/ou une machine de remplissage de contenants.

14. Procédé (100) de commande d'un dispositif de traitement (10) selon l'une quelconque des revendications précédentes, dans lequel le dispositif de séparation (24) passe dans le deuxième état de fonctionnement (106) lorsque le premier dispositif de mesure (16) émet une valeur de mesure de concentration qui est supérieure à une première valeur de seuil prédéfinie (27) et passe dans le premier état de fonctionnement (112) lorsque le deuxième dispositif de mesure (20) émet une valeur de mesure de concentration, qui est inférieure à une deuxième valeur de seuil prédéfinie (27).

15. Procédé (100) selon la revendication 14, **caractérisé en ce qu'**un dispositif de commande (30) du dispositif de traitement (10) pour commander le dispositif de traitement (10), en particulier pour des verrouillages de porte, une évacuation par aspiration, une ventilation, un arrêt de production et/ou une libération de production, prend en compte (102) des valeurs de mesure du premier dispositif de mesure (16), lorsque le dispositif de séparation (24) est dans le premier état de fonctionnement, et prend en compte (108) des valeurs de mesure du deuxième dispositif de mesure (20), lorsque le dispositif de séparation (24) est dans le deuxième état de fonctionnement.
